# EUROPEAN PATENT APPLICATION

(11) **EP 3 401 015 A1**
(43) Date of publication of application: **14.11.2018**
(21) Application number: 16884022.1
(22) Date of filing: 21.12.2016
(51) Int. Cl.: B01L 7/00, C12Q 1/68, G01N 21/552

(54) **SURFACE MEASUREMENT SENSING-BASED REAL TIME NUCLEIC ACID AMPLIFICATION MEASURING DEVICE**

(30) Priority: 08.01.2016 KR 20160002418
(71) Applicant: Korea University Research and Business Foundation, Seoul 02841 (KR)
(72) Inventor: SHIN, Se-Hyun, Seoul 06574 (KR); NA, Won-Hwi, Seoul 05507 (KR); JANG, Dae-Ho, Ansan-si Gyeonggi-do 15483 (KR)
(74) Representative: RatnerPrestia
(86) International application number: PCT/KR2016/014981
(87) International publication number: WO 2017/119639

(57) **Abstract**

Disclosed is a real-time nucleic acid amplification measurement apparatus using a surface measurement technique, the apparatus including: a microfluidic chip having a closed loop shaped-microfluidic channel; a sample injecting and closing part communicating with the microfluidic channel, and operating in an injecting mode in which a reaction sample is injected to the microfluidic channel or operating in a closed mode in which the microfluidic channel forms a closed-loop in a state that the reaction sample has been injected to the microfluidic channel; a fluid movement generating part inducing the reaction sample to circulate inside the microfluidic channel; a plurality of heating parts individually heating a plurality of heating areas with different temperatures to amplify nucleic acid in the reaction sample; and a surface measurement part detecting the nucleic acid in the reaction sample at a predetermined area inside the microfluidic channel.

## Description

### Technical Field

The present invention relates to a real-time nucleic acid amplification measurement apparatus using a surface measurement technique. More specifically, the present invention relates to a real-time nucleic acid amplification measurement apparatus using a surface measurement technique, the apparatus being capable of detecting an amplification process of nucleic acid in real-time, based on the surface measurement technique such as surface plasmon resonance (SPR).

### Background Art

A real-time polymerase chain reaction (hereinafter, real-time PCR) has been widely utilized in recent years for performing nucleic acid analysis, since PCR is capable of checking a nucleic acid amplification product in real-time during a reaction cycle without performing electrophoresis in a gel and capable of conducting quantitative analysis.

In general, an apparatus for implementing the real-time PCR includes a thermal cycler provided with at least one heating block performing a nucleic acid amplification reaction, and a sensor/detector detecting a signal generated from a nucleic acid amplification product in real-time.

In recent years in the medical field, efficient diagnoses and therapeutic methods for implementing personalized medicine have been actively developed. In order to achieve personalized medicine practically, rapid and accurate diagnosis and treatment for a number of objects is necessary. In this case of the diagnosis and treatment, the nucleic acid amplification step is a basic precondition step, and the real-time PCR, which is one example of performing the step, is an essential in implementing personalized medicine.

However, real-time PCR is a complicated process that it takes significant time to complete. In addition, an apparatus for performing real-time PCR is generally expensive, large, and is capable of measuring only one or three to four diagnostic markers in one reaction tube, whereby it is difficult to realize practical personalized medicine.

Korean Patent Application Publication No. 10-2004-0048754 discloses a temperature controlled real-time fluorescence detection apparatus, which is a portable and compact fluorescence detection apparatus. The apparatus is capable of sensitively detecting fluorescence of several wavelengths among hundreds to thousands of samples in a few seconds even for low concentration of the samples, searching and analyzing enzyme reactions in real-time, and being provided at a low price.

In addition, Korean Patent No. 10-0794703 discloses a real-time monitoring apparatus for biochemical reaction, and a technique thereof is capable of comparatively analyzing reaction degrees of various kinds of samples during reactions in a reaction tube plate by minimizing an optical detection sensitivity variation.

In the real-time PCR in the related art, a reagent, which is designed to fluoresce in an amplified nucleic acid as the nucleic acid is amplified, is generally used and the amount of fluorescence is measured at each end of a cycle such that the amount of genes initially existing in the sample is estimated by using time from start to until the amplification occurs.

However, the reagent designed to fluoresce is very expensive, thus the cost for diagnosis is increased. In addition, the real-time PCR device uses a heating block having a large heat capacity, as conventional PCRs do, such that time required to control temperature is long, leading to long measurement time.

Furthermore, a real-time PCR method in the related art requires substantial time and manpower until actual measurement results are obtained because the reagent to be put in the equipment is manually put into a PCR tube by the experimenter. In addition, multiple detections in a single tube are limited due to limitation of fluorescent sample or fluorescence measurement wavelength.

### Disclosure

### Technical Problem

Accordingly, the present invention has been made keeping in mind the above problems occurring in the related art, and an object of the present invention is to provide a real-time nucleic acid amplification measurement apparatus using a surface measurement technique in which a microfluidic chip applied with a microfluidic technology for polymerase chain reaction (PCR) and a surface measurement technique such as surface plasmon resonance (SPR) are applied whereby product cost can be lowered significantly, use of a reagent is eliminated or minimized whereby cost for measurement can be lowered significantly, and a receptor array is formed on a surface of a sensor chip whereby hundreds to thousands of biomarkers can be measured simultaneously.

In addition, another object of the present invention is to provide a real-time nucleic acid amplification measurement apparatus using a surface measurement technique, the apparatus capable of improving reliability of measurement by eliminating measurement errors caused by bubbles that may occur during a process of injecting a reaction sample into the microfluidic chip or during measurement process.

In addition, still another object of the present invention is to provide a real-time nucleic acid amplification measurement apparatus using a surface measurement technique, the apparatus capable of performing quantitative analysis as well as qualitative analysis in real-time measurement.

### Technical Solution

In order to accomplish the above objects, the present invention provides a real-time nucleic acid amplification measurement apparatus using a surface measurement technique, the apparatus including: a microfluidic chip having a closed loop shaped-microfluidic channel; a sample injecting and closing part communicating with the microfluidic channel, and operating in an injecting mode in which a reaction sample is injected to the microfluidic channel or operating in a closed mode in which the microfluidic channel forms a closed-loop in a state that the reaction sample has been injected to the microfluidic channel; a fluid movement generating part inducing the reaction sample to circulate inside the microfluidic channel; a plurality of heating parts individually heating a plurality of heating areas with different temperatures to amplify nucleic acid in the reaction sample; and a surface measurement part detecting the nucleic acid in the reaction sample at a predetermined area inside the microfluidic channel.

Meanwhile, in order to accomplish the above objects, the present invention provides a real-time nucleic acid amplification measurement apparatus using a surface measurement technique according to another embodiment, the apparatus including: a microfluidic chip provided with a microfluidic channel, and a first sample buffer chamber and a second sample buffer chamber, which are provided at both sides of the microfluidic channel respectively; a fluid movement generating part inducing the reaction sample to oscillatingly flow in the first buffer chamber, the microfluidic channel, and the second buffer chamber; a surface measurement part detecting nucleic acid in the reaction sample at a middle area of a flow direction in the microfluidic channel; and a first nucleic acid amplification part and a second nucleic acid amplification part each provided at both sides of the surface measurement part and each provided with a plurality of heating parts individually heating a plurality of heated areas with different temperatures to amplify the nucleic acid of the reaction sample repeatedly flowing in the microfluidic channel.

Here, the plurality of heated areas may fall into a denaturation area, an annealing area, and an extension area, and the heating parts may include a denaturation heating part, an annealing heating part, and an extension heating part respectively heating the denaturation area, the annealing area, and the extension area to amplify nucleic acid by polymerase chain reaction (PCR).

In addition, the first nucleic acid amplification part and the second nucleic acid amplification part may be arranged in an order of the denaturation heating part, the annealing heating part, the extension heating part, the annealing heating part, and the denaturation heating part.

In addition, the fluid movement generating part may be provided in a type of a syringe pump.

In addition, the surface measurement part may include a surface plasmon resonance (SPR) sensing part using a SPR phenomenon.

Here, the SPR sensing part may include: a metal thin film chip provided at an inner surface of the microfluidic channel to detect plasmon reaction; a coupler provided at outside the metal thin film chip; a light source irradiating the metal thin film chip with measurement light from outside the microfluidic chip; and a light receiver receiving light reflected by the metal thin film chip.

In addition, the SPR sensing part may be provided with one method among a variable-angle SPR method, a variable-wavelength SPR method, and an SPR imaging method.

In addition, the surface measurement part may include: a nanoplasmonic sensor chip provided at an inner surface of the microfluidic channel and on whose surface nano metal particles generating nanoplasmonic effect are solidified; a light source irradiating the nanoplasmonic sensor chip with measurement light from outside the microfluidic channel to induce the nano metal particles to generate a nanoplasmonic effect; and a light receiver receiving a light transmitted through the nanoplasmonic sensor chip, wherein the surface measurement part may detect nucleic acid by using at least one among a wavelength and an intensity of the transmitted light.

In addition, the surface measurement part may include: a quartz crystal microbalance (QCM) sensor provided inside the microfluidic channel; and a high frequency power supply applying high frequency power to the QCM sensor, wherein the surface measurement part may detect nucleic acid by using a frequency change according to a mass change of the QCM sensor due to nucleic acid adhered to a surface thereof.

In addition, the surface measurement part may include: a first electrode provided at an inner surface of the microfluidic channel and having a characteristic that combines with nucleic acid; a second electrode provided at the inner surface of the microfluidic channel and having a characteristic that does not combine with nucleic acid; a third electrode applied with a different polarity with the first electrode and the second electrode; and a measurement voltage supply applying same polarity of voltage to the first electrode and the second electrode, and applying voltage to the third electrode, the voltage having opposite polarity to the first electrode and the second electrode, wherein the surface measurement part may detect nucleic acid by using a current value according to a conductivity change while the measurement voltage supply alternately switches between positive voltage to negative voltage.

In addition, the plurality of heating parts may fall into a denaturation area, an annealing area, and an extension area, and the heating parts may include a denaturation heating part, an annealing heating part, and an extension heating part respectively heating the denaturation area, the annealing area, and the extension area to amplify nucleic acid by polymerase chain reaction (PCR).

In addition, the sample injecting and closing part may include: a four-way valve including a first connecting port, a second connecting port, a third connecting port, and a fourth connecting port, wherein the first connecting port and the second connecting port are connected to the microfluidic channel and the microfluidic channel forms the closed-loop if the first connecting port and the second connecting port are connected to each other; a sample inlet having one end through which the reaction sample is injected and a remaining end is connected to the third connecting port of the four-way valve; and a sample outlet having one end is connected to the fourth connecting port of the four-way valve; wherein, in the injecting mode, the four-way valve connects the first connecting port and the third connecting port to each other and connects the second connecting port and the fourth connecting port such that the reaction sample injected through the sample inlet is allowed to flow into the microfluidic channel, and in the closed mode, the four-way valve connects the first connecting port and the second connecting port to each other such that the microfluidic channel forms the closed-loop.

In addition, the sample injecting and closing part may further include a gas discharging membrane provided at an end of the sample outlet and allowing gas to pass therethrough and blocking liquid, wherein the reaction sample injected into the microfluidic channel in the injecting mode may pass the second connecting port, the microfluidic channel, and the fourth connecting port and flows to the sample outlet, and gas inside the reaction sample may be discharged to an outside of the microfluidic channel through the gas discharging membrane while being blocked by the gas discharging membrane.

In addition, the apparatus may further include: a gas discharging portion provided at least at a portion of an upper surface of the microfluidic channel in terms of the gravity direction, and made of a material that allows gas to pass therethrough and blocks liquid; and a gas discharging vacuum portion applying a vacuum pressure to the gas discharging portion to discharge bubbles through the gas discharging portion, the bubbles generated in a heating process by the heating parts.

The plurality of heating parts may constitute the fluid movement generating part in which the microfluidic chip is disposed in the gravity direction such that the reaction sample in the microfluidic channel flows in the gravity direction, the plurality of heating parts is disposed in the gravity direction and arranged in an order of decreasing temperature, and a density change of the reaction sample heated by the plurality of the heating parts disposed in the gravity direction causes heat convection whereby the reaction sample circulates.

Here, the fluid movement generating part may include: a pump member made of an elastic material and constituting a wall surface of a area of the microfluidic channel; and a pump drive pumping the pump member such that the reaction sample flows inside the microfluidic channel.

In addition, the fluid movement generating part may include: an impeller operating to allow the reaction sample to flow in the microfluidic channel; and an impeller drive disposed outside the microfluidic channel and driving the impeller by magnetic force.

In addition, the fluid movement generating part may include an acoustic wave generator generating a high-frequency sound wave in a flow direction of reaction sample to allow the reaction sample to flow.

In addition, the fluid movement generating part may include: a plurality of dielectrophoretic electrodes arranged at an inner wall of the microfluidic channel in a flow direction of the reaction sample; and a power supply for the dielectrophoretic electrodes, the power supply supplying power to cause a flow of the reaction sample by dielectrophoresis.

Here, the fluid movement generating part may further include a laser emitting part irradiating one of the dielectrophoretic electrodes, wherein the flow of the reaction sample and vortex may occur in a direction starting from the irradiated dielectrophoretic electrode to another dielectrophoretic electrode.

Here, the metal thin film chip may be provided with a dextran-based or polymer-based three-dimensional surface material on a surface thereof to increase a sensing surface area, and provided with a receptor for reaction with nucleic acid on the three-dimensional surface material.

In addition, an inner wall surface of the microfluidic channel provided above the metal thin film chip may be configured to protrude toward the metal thin film chip such that a portion of the channel provided with the metal thin film chip is configured to become narrow.

In addition, the apparatus may further include: a protruding part protruding toward the metal thin film chip from an inner wall surface of the microfluidic channel provided above the metal thin film chip such that a portion of the channel provided with the metal thin film chip is configured to become narrow, wherein the protruding part may be provided with a micro pattern inducing mix of the reaction sample flowing inside the microfluidic channel.

### Advantageous Effects

According to above-described configuration of the present invention, product cost can be lowered significantly by applying a microfluidic chip applied with a microfluidic technology for polymerase chain reaction (PCR) and a surface measurement technique such as surface plasmon resonance

(SPR) . In addition, cost for measurement can be lowered significantly by eliminating or minimizing use of a reagent.

In addition, reliability of measurement can be improved by eliminating measurement errors caused by bubbles that may occur during a process of injecting a reaction sample into the microfluidic chip or during a measurement process.

Furthermore, quantitative analysis as well as qualitative analysis can be performed in real-time measurement.

### Description of Drawings

FIG. 1 is a perspective view showing a real-time nucleic acid amplification measurement apparatus using a surface measurement technique according to a first embodiment of the present invention;
FIG. 2 is a plan view showing the real-time nucleic acid amplification measurement apparatus according to the first embodiment of the present invention;
FIG. 3 depicts diagrams showing operations of a sample injecting and closing part of the real-time nucleic acid amplification measurement apparatus according to the first embodiment of the present invention;
FIG. 4 depicts diagrams showing examples for a fluid movement generating part of the real-time nucleic acid amplification measurement apparatus according to the first embodiment of the present invention;
FIGS. 5 to 9 depict diagrams each showing an example for a configuration of a surface measurement part of the real-time nucleic acid amplification measurement apparatus according to the first embodiment of the present invention;
FIG. 10 is a diagram showing a gas discharging portion and a gas discharging vacuum portion according to the present invention; and
FIG. 11 is a diagram showing a real-time nucleic acid amplification measurement apparatus using a real-time surface sensing technique according to a second embodiment of the present invention.

### Best Mode

The present invention relates to a real-time nucleic acid amplification measurement apparatus using a surface measurement technique, the apparatus including: a microfluidic chip having a closed loop shaped-microfluidic channel; a sample injecting and closing part communicating with the microfluidic channel, and operating in an injecting mode in which a reaction sample is injected to the microfluidic channel or operating in a closed mode in which the microfluidic channel forms a closed-loop in a state that the reaction sample has been injected to the microfluidic channel; a fluid movement generating part inducing the reaction sample to circulate inside the microfluidic channel; a plurality of heating parts individually heating a plurality of heating areas with different temperatures to amplify nucleic acid in the reaction sample; and a surface measurement part detecting the nucleic acid in the reaction sample at a predetermined area inside the microfluidic channel.

### Mode for Invention

Hereinbelow, various embodiments of the present invention will be described in detail with reference to the accompanying drawings.

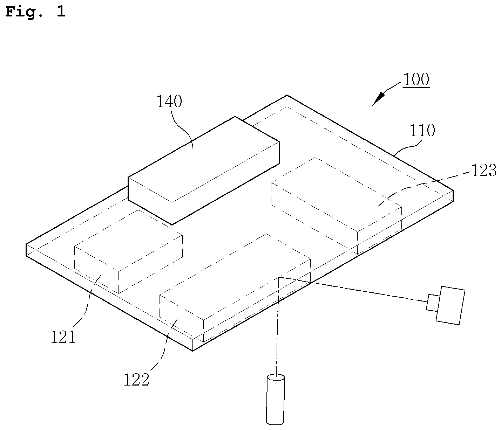

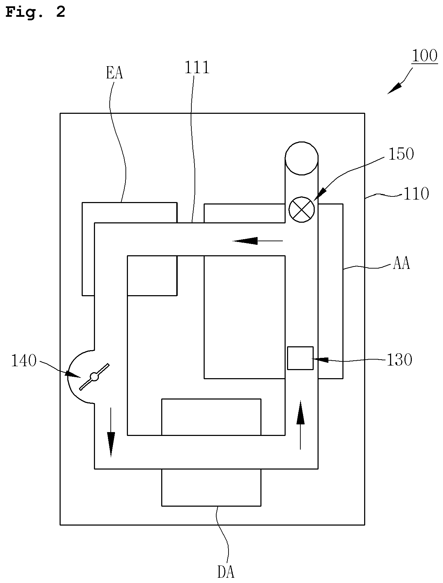

FIG. 1 is a perspective view showing a real-time nucleic acid amplification measurement apparatus using a surface measurement technique 100 and FIG. 2 is a plan view showing the real-time nucleic acid amplification measurement apparatus using the real-time surface sensing technique 100.

Referring to FIGS. 1 and 2, the real-time nucleic acid amplification measurement apparatus using the real-time surface sensing technique 100 (hereinafter, referred to as a real-time nucleic acid amplification measuring apparatus 100) according to the first embodiment of the present invention includes a microfluidic chip 110, a sample injecting and closing part 150, a fluid movement generating part 140, a plurality of heating parts 121, 122, and 123, and a surface measurement part 130.

The microfluidic chip 110 is provided with a closed loop shaped-microfluidic channel 111 as shown in FIG. 2. A reaction sample, which is a subject to be measured, flows in the microfluidic channel 111. The microfluidic channel 111 is provided as a transparent material such that the surface measurement part 130 detects a nucleic acid of the reaction sample flowing in the microfluidic channel 111.

The sample injecting and closing part 150 operates in an injecting mode and in a closed mode. After the reaction sample is injected, the microfluidic channel 111 forms a closed-loop. Specifically, the sample injecting and closing part 150 is configured to communicate with the microfluidic channel 111 and operates in the injecting mode in which the reaction sample is injected to the microfluidic channel 111. In addition, after injecting the reaction sample to the microfluidic channel 111, the sample injecting and closing part 150 operates in the closed mode in which the microfluidic channel 111 forms the closed-loop.

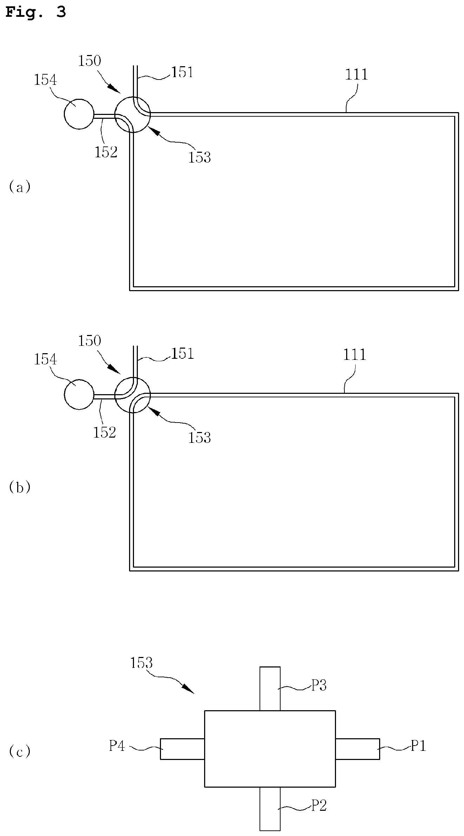

FIG. 3 depicts diagrams showing operations of the sample injecting and closing part 150 of the real-time nucleic acid amplification measuring apparatus 100 according to the first embodiment of the present invention. In the embodiment shown in FIG. 3, the sample injecting and closing part 150 according to the present invention is provided with a four-way valve 153, a sample inlet 151, and a sample outlet 152.

As shown in FIG. 3(c), the four-way valve 153 includes four connecting ports: a first connecting port P1, a second connecting port P2, a third connecting port P3, and a fourth connecting port P4. The first connecting port P1 and the second connecting port P2 are disposed between ends of the microfluidic channel 111 such that when the first connecting port P1 and the second connecting port P2 are connected to each end of the microfluidic channel 111, the microfluidic channel 111 forms the closed-loop. The third connecting port P3 and the fourth connecting port P4 are connected to the sample inlet 151 and the sample outlet 152, respectively.

The reaction sample is injected through one end of the sample inlet 151, and an opposite end thereof is connected to the third connecting port P3 of the four-way valve 153 as described above. In addition, the sample outlet 152 is connected to the fourth connecting port P4 of the four-way valve 153.

Through the above-described configuration, the four-way valve 153 in the injecting mode as shown in FIG. 3(a) connects the first connecting port P1 and the third connecting port P3 to each other and connects the second connecting port P2 and the fourth connecting port P4 to each other such that the reaction sample injected through the sample inlet 151 is allowed to flow into the microfluidic channel 111. That is, the reaction sample injected through the sample inlet 151 passes the third connecting port P3 and the first connecting port P1 to flow in the microfluidic channel 111. Then, the reaction sample passes the second connecting port P2 and the fourth connecting port P4 and flows to the sample outlet 152 such that the microfluidic channel 111 is filled with the reaction sample.

Here, the sample injecting and closing part 150 according to the present invention may be provided with the four-way valve 153 provided at an end of the sample outlet 152. The four-way valve 153 may be made of a material that allows gas to pass therethrough and blocks liquid. Accordingly, when the reaction sample is injected into the microfluidic channel 111 and the microfluidic channel 111 filled with the reaction sample is pressurized continuously in the direction of injection, air or bubbles remaining in the microfluidic channel 111 are discharged through the four-way valve 153 whereby deterioration of accuracy of the measurement due to bubbles and the like in the nucleic acid detection can be prevented.

As described above, after the microfluidic channel 111 is filled with the reaction sample in the state where the bubbles and the like are removed, when the four-way valve 153 operates in the closed mode in which the first connecting port P1 and the second connecting port P2 are connected to each other and the third connecting port P3 and the fourth connecting port P4 are connected to each other, the microfluidic channel 111 forms the closed-loop as shown in FIG. 3(b).

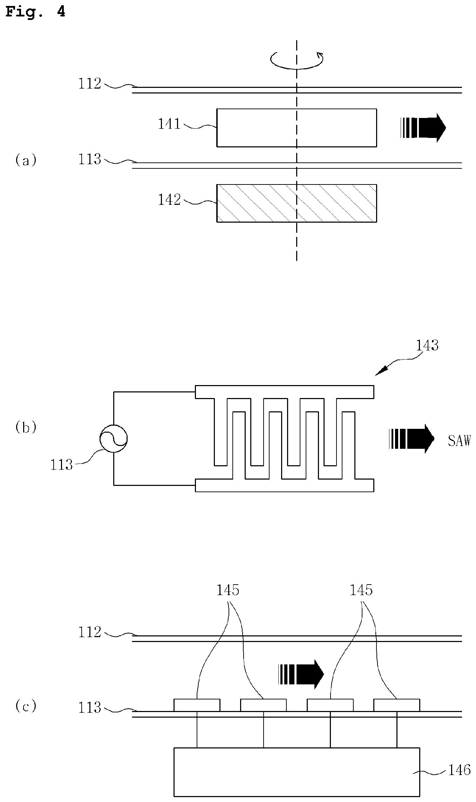

Meanwhile, the fluid movement generating part 140 induces the reaction sample to circulate inside the microfluidic channel 111. FIG. 4 depicts diagrams showing examples for the fluid movement generating part 140.

As shown in FIG. 4(a), the fluid movement generating part 140 according to an embodiment of the present invention may include an impeller 141 and an impeller drive 142. The impeller 141 operates to allow the reaction sample to flow in the microfluidic channel 111. For example, the impeller 141 rotates or vibrates to allow the reaction sample to flow. The impeller drive 142 drives the impeller 141 at an outside of the microfluidic channel 111. In the present invention, the impeller 141 is provided as a magnetic body, and the impeller drive 142, which is disposed outside the microfluidic channel 111, drives the impeller 141 by magnetic force.

The fluid movement generating part 140 according to another embodiment of the present invention may include an acoustic wave generator. The acoustic wave generator generates a high-frequency sound wave in a flow direction of the reaction sample to allow the reaction sample to flow. Referring to FIG. 4(b), when an interdigital transducer (IDT) 143 is powered by an IDT power supply 144 while the IDT 143 is disposed on a bottom surface 113 of the microfluidic channel 111, a surface acoustic wave (SAW) is generated by a piezoelectric effect such that the reaction sample flows by the SAW.

The fluid movement generating part 140 according to still another embodiment of the present invention may induce the movement of the reaction sample by dielectrophoresis. Referring to FIG. 4(c), the fluid movement generating part may include a plurality of dielectrophoretic electrodes 145 arranged at an inner wall of the microfluidic channel 111 in a flow direction of the reaction sample and a power supply 146 supplying power to the dielectrophoretic electrodes.

The power supply 146 for the dielectrophoretic electrodes supplies power to the plurality of dielectrophoretic electrodes 145 in a predetermined pattern to induce dielectrophoresis, and an electric field generated between a pair of the dielectrophoretic electrodes 145 causes the flow of the reaction sample.

Here, the fluid movement generating part 140 may further include a laser emitting part irradiating one of the dielectrophoretic electrodes 145 with laser, in addition to dielectrophoresis. The movement of the reaction sample and vortex occur in a direction starting from the one of the dielectrophoretic electrodes 145 irradiated by the laser emitting part to other dielectrophoretic electrodes 145 such that the movement of the reaction sample and mix of the nucleic acid therein can occur at the same time.

Besides, the fluid movement generating part 140 may include a pump member (not shown) made of an elastic material and constituting a wall surface of a area of the microfluidic channel 111, and a pump drive (not shown) pumping the pump member such that the reaction sample flows inside the microfluidic channel 111. That is, the wall surface of the microfluidic channel 111 is made of the elastic material and is vibrates by the pump drive to cause the pumping effect such that the reaction sample is induced to move.

Meanwhile, the plurality of heating parts 121, 122, and 123 heat a plurality of heated areas of the heat microfluidic channel 111 with different temperatures individually to amplify the nucleic acid of the reaction sample. In the present invention, it is exemplified that the plurality of heating parts 121, 122, and 123 is configured to amplify nucleic acid by polymerase chain reaction.

A detailed description will be described with reference to FIGS. 1 and 2. As shown in FIG. 2, it is exemplified that the plurality of heated areas according to the present invention falls into an annealing area AA, an extension area EA, and a denaturation area DA.

In addition, the heating parts 121, 122, and 123 include an annealing heating part 122 heating the annealing area AA, an extension heating part 121 heating the extension area EA, and a denaturation heating part 123 heating the denaturation area. The annealing heating part 122 heats the annealing area AA such that the reaction sample is heated to about 50°C, the extension heating part 121 heats the extension area EA such that the reaction sample is heated to about 72°C, and the denaturation heating part 123 heats the denaturation area such that the reaction sample is heated to about 95°C.

According to the above-described configuration, the nucleic acid of the reaction sample accommodated in the microfluidic channel 111 forming the closed loop is amplified by being heated to predetermined temperatures while sequentially passing through the denaturation area, the annealing area AA, and the extension area EA, such that it is continuously amplified in the continuous circulation and the amount of the nucleic acid is increased.

As described above, the nucleic acid of the reaction sample circulated inside the microfluidic channel 111 is detected in real-time by the surface measurement part 130. The surface measurement part 130 detects the nucleic acid of the reaction sample at a predetermined area inside the microfluidic channel 111.

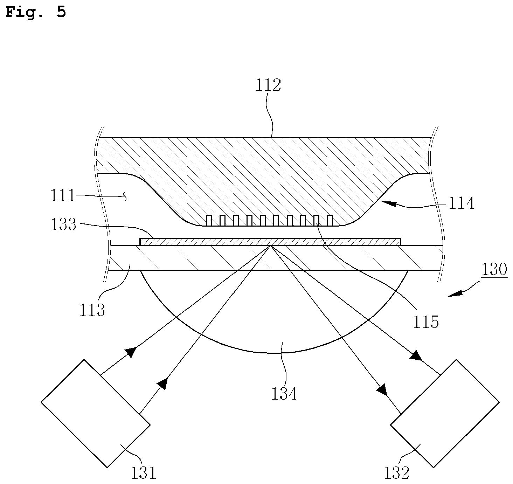

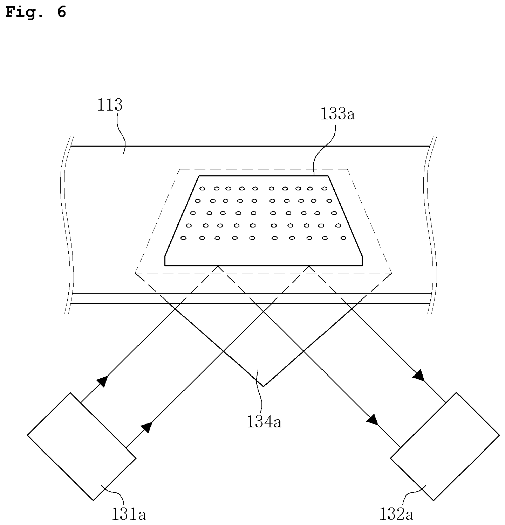

FIGS. 5 to 9 depict diagrams each showing an example for a configuration of the surface measurement part. The surface measurement parts 130 and 130a shown in FIGS. 5 and 6 depict configurations of surface plasmon resonance (SPR) sensing parts 130 and 130a using a SPR phenomenon.

As shown in FIGS. 5 and 6, the SPR sensing parts 130 and 130a are provided with metal thin film chips 133 and 133a, couplers 134 and 134a, light sources and 131a, and light receivers 132 and 132a, respectively. Each of the metal thin film chips 133 and 133a is provided at an inner surface of the microfluidic channel 111 to detect a plasmon reaction. In the present invention, it is exemplified that each of the metal thin film chips 133 and 133a is provided with a dextran-based or a polymer-based three-dimensional surface material on each surface thereof to increase each sensing surface area, and provided with a receptor for reaction with the nucleic acid on the three-dimensional surface material.

Each of the couplers 134 and 134a is provided at each outside of the metal thin film chips 133 and 133a. In the embodiment of FIGS. 5 and 6, each of the couplers 134 and 134a faces each of the metal thin film chips 133 and 133a with the bottom surface 113 of the microfluidic chip 110 therebetween, the bottom surface 113 serving to form the microfluidic channel 111. However, it is also possible that the each of the couplers 134 and 134a and each of the metal thin film chips 133 and 133b are attached directly and serve to form a part of the bottom surface 113.

From outside the microfluidic chip 110, the light sources 131 and 131a irradiate the metal thin film chips 133 and 133a with measurement light. In FIG. 5, it is exemplified that the light source 131 has a variable-angle structure for emitting a light. On the other hand, in FIG. 6, it is exemplified that the light source 131a has a structure for emitting parallel light. That is, the embodiment shown in FIG. 5 applies the variable-angle SPR method, and the embodiment shown in FIG. 6 applies an SPR imaging method. Here, it is obvious that a variable-wavelength SPR method may be applied for the SPR method.

The light receivers 132 and 132a receive a reflection light reflected by the metal thin film chips 133 and 133a, respectively. Then, the reflection light received by the light receivers 132 and 133a is analyzed such that the nucleic acid detected by the metal thin film chips 133 and 133a is measured.

As shown in FIG. 5, each inner wall surface of the microfluidic channel 111 provided above the metal thin film chips 133 and 133a is configured with a protruding part 114 protruding toward the metal thin film chips 133 and 133a such that each portion of the channel provided with the metal thin film chips 133 and 133a is configured to become narrow. In addition, the protruding part 114 may be provided with a micro pattern 115 that induces mix of the reaction sample flowing inside the microfluidic channel 111. Accordingly, the reaction sample is allowed to flow closer to the metal thin film chips 133 and 133a on top of the metal thin film chips 133 and 133a. Thus, the reaction sample is continuously mixed by the micro pattern 115 and supplied to the metal thin film chips 133 and 133a at a uniform concentration such that the nucleic acid can be more easily attached to the metal thin film chips 133 and 133a.

Here, although the protruding part 114 in which the microfluidic channel 111 is narrowed is not shown in the embodiments of FIGS. 6 to 9 which will be described later, it is obvious that the configuration in which the microfluidic channel 111 is narrowed may be applied to the embodiments of FIGS. 6 to 9.

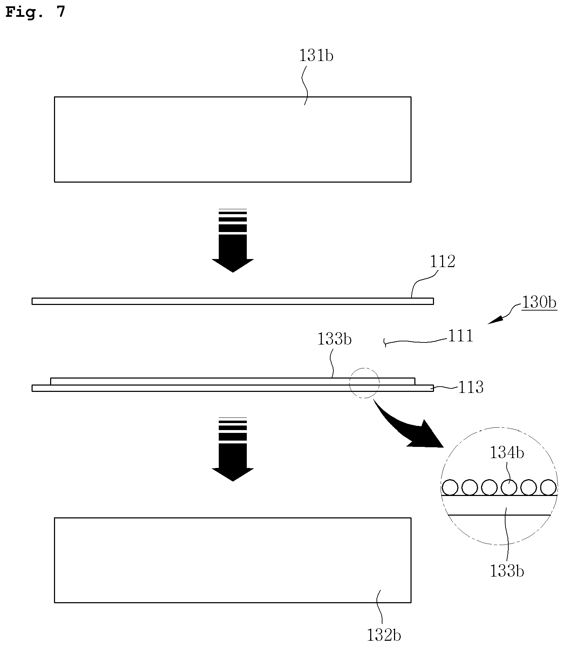

FIG. 7 shows an example of a surface measurement part 130b according to another embodiment of the present invention in which the nucleic acid is measured by using a nanoplasmonic effect. Specifically, the surface measurement part 130b may include a nanoplasmonic sensor chip 133b, a light source 131b, and a light receiver 132b.

The nanoplasmonic sensor chip 133b is provided at an inner surface of the microfluidic channel 111. In the present invention, it is exemplified that the nanoplasmonic sensor chip 133b is provided at the bottom surface 113 serving to form the microfluidic channel 111. Nano metal particles 134b that generate a nanoplasmonic effect are solidified on a surface of the nanoplasmonic sensor chip 133b.

From outside the microfluidic channel 111, the light source 131b irradiates the nanoplasmonic sensor chip 133b with measurement light to induce the nano metal particles 134b to generate a nanoplasmonic effect. Then, the light receiver 132b receives a light transmitted through the nanoplasmonic sensor chip 133b.

In the present invention, the light source 131b irradiates the nanoplasmonic sensor chip 133b with measurement light from an outside of an upper portion of the microfluidic channel 111 toward an upper surface 112 of the microfluidic channel 111. It is exemplified that the light receiver 132b receives the light transmitted through the nanoplasmonic sensor chip 133b and the bottom surface 113 at an outside of a bottom portion of the microfluidic channel 111. Here, the nucleic acid can be measured by analyzing the transmitted light received by the light receiver 132b, in detail, the nucleic acid can be detected by using at least one among a wavelength and an intensity of the transmitted light.

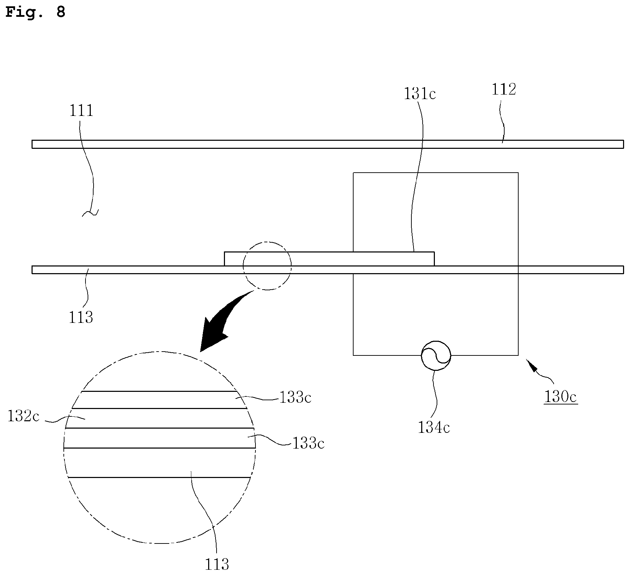

FIG. 8 shows an example of a surface measurement part 130c according to still another embodiment of the present invention in which quartz crystal microbalance (QCM) is used. Referring to FIG. 8, the surface measurement part 130c may include a QCM sensor 131c and a high frequency power supply 134c.

The QCM sensor 131c is provided inside the microfluidic channel 111. The QCM sensor 131c is provided with a crystal resonator 132c varying in length due to an internal crystal structure varying when applied with voltage, and a pair of electrodes 133c attached to the crystal resonator to apply voltage to the crystal resonator.

When the high frequency power supply 134c applies high frequency power to the QCM sensor 131c, the surface measurement part 130 senses a frequency change due to a mass change of the QCM sensor 131c and thus detects the nucleic acid.

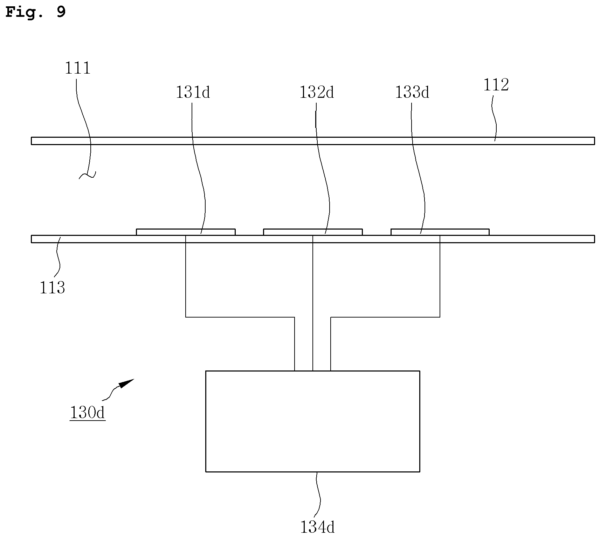

FIG. 9 shows an example of a surface measurement part 130d according to still another embodiment of the present invention in which an electrochemical sensing method is used. Referring to FIG. 9, the surface measurement part 130d may include a first electrode 131d, a second electrode, a third electrode 133d, and a measurement voltage supply 134d.

The first electrode 131d is provided at an inner surface of the microfluidic channel 111, for example, at the bottom surface 113, and has a characteristic wherein the nucleic acid adheres to the first electrode 131d. The second electrode is provided at the inner surface of the microfluidic channel 111 while spaced apart with the first electrode 131d, and has a characteristic wherein the nucleic acid does not adhere to the second electrode. The third electrode 133d is applied with a different polarity with the first electrode 131d and the second electrode 132d.

The measurement voltage supply 134d applies measurement voltage to the first electrode 131d, the second electrode 132d, and the third electrode 133d. In detail, the measurement voltage supply 134d applies same polarity of voltage to the first electrode 131d and the second electrode 132d, and applies opposite polarity of voltage to the third electrode 133d, different to the first electrode 131d and the second electrode 132d.

According to the above-described configuration, the surface measurement part 130 detects the nucleic acid by using a current value according to a conductivity change while the measurement voltage supply 134d alternately switches between positive voltage to negative voltage.

According to the above-described configuration, the real-time nucleic acid amplification measuring apparatus 100 according to the present invention applies the microfluidic chip 110 provided with the microfluidic channel 111 and a surface measurement technique such as SPR whereby product cost can be lowered significantly, and use of a reagent is eliminated or minimized whereby cost for measurement can be lowered significantly.

In addition, the real-time nucleic acid amplification measuring apparatus 100 according to the present invention applies the four-way valve 153 to remove gas such as bubbles being generated or remaining in the microfluidic channel 111 during the injection of the reaction sample, whereby reliability of the measurement can be improved.

In addition, during the process that the nucleic acid is amplified while the reaction sample circulates in the closed loop shaped-microfluidic channel 111, the amount of the nucleic acid can be measured in real-time, whereby the amplification of the nucleic acid can be analyzed quantitatively in real-time.

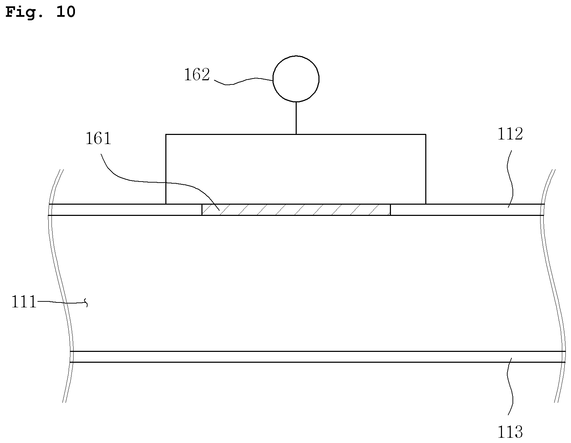

Meanwhile, the real-time nucleic acid amplification measuring apparatus 100 according to the present invention may include a gas discharging portion 161 and a gas discharging vacuum portion 162. Referring to FIG. 10, the gas discharging portion 161 is provided at least at a portion of the upper surface of the microfluidic channel 111 in terms of the gravity direction. In FIG. 10, it is exemplified that a portion of the upper surface 112 of the microfluidic channel 111 is formed as the gas discharging portion 161.

The gas discharging portion 161 is made of a material that allows gas to pass therethrough and blocks liquid. Accordingly, the bubbles remaining in the reaction sample or the bubbles generated in the heating process by the heating parts 121, 122, and 123 can be discharged through the gas discharging portion 161. In addition, the gas discharging vacuum portion 162 applies a vacuum pressure to the gas discharging portion 161 to smooth the discharge of gas such as bubbles.

In the present invention, it is exemplified that the gas discharging portion 161 is provided at a rear end of the heating parts 121, 122, and 123 based on a circulation direction of the reaction sample. In particular, it is preferable that the gas discharging portion 161 is provided at a rear end of the denaturation heating part 123 which is heated to the highest temperature among the heating parts 121, 122, and 123.

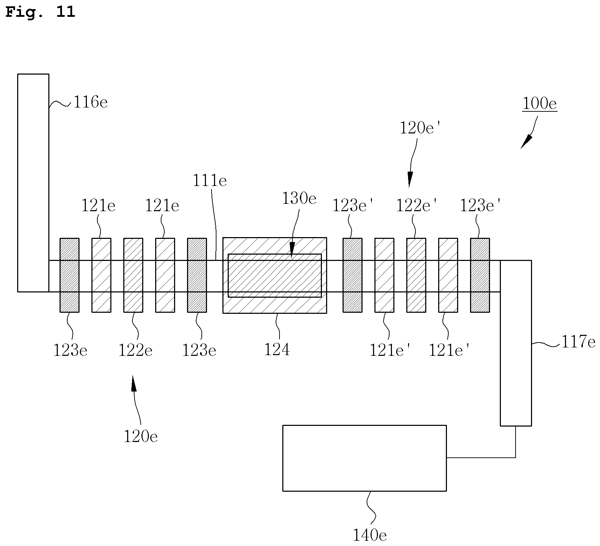

Hereinafter, a real-time nucleic acid amplification measuring apparatus 100e according to a second embodiment of the present invention will be described in detail with reference to FIGS. 11.

The real-time nucleic acid amplification measuring apparatus 100e according to the second embodiment of the present invention includes a microfluidic chip (not shown, hereinafter, the same applies), a fluid movement generating part 140e, a surface measurement part 130e, a first nucleic acid amplification part 120e, and a second nucleic acid amplification part 120e'.

The microfluidic chip is provided with a microfluidic channel 111e, a first buffer chamber 116e for sample, and a second buffer chamber 117e for sample. As shown in FIG. 11, the microfluidic channel 111e is configured to be a straight line in the microfluidic chip.

The first buffer chamber 116e and the second buffer chamber 117e are provided at both sides of the microfluidic channel 111e respectively. The first buffer chamber 116e, the microfluidic channel 111e, and the second buffer chamber 117e are configured to communicate with each other.

The fluid movement generating part 140e induces the reaction sample to oscillatingly flows in the first buffer chamber 116e, the microfluidic channel 111e, and the second buffer chamber 117e, repeatedly. In the second embodiment of the present invention, it is exemplified that fluid movement generating part 140e is provided in a type of a syringe pump.

The surface measurement part 130e detects the nucleic acid of the reaction sample at a middle area of the flow direction in the microfluidic channel 111e. The surface measurement part 130e according to the second embodiment of the present invention may be applied with the various examples described in the first embodiment and thus a detailed description will be omitted.

The first nucleic acid amplification part 120e and the second nucleic acid amplification part 120e' are provided at both sides of the surface measurement part 130e respectively. In addition, each of the first nucleic acid amplification part 120e and the second nucleic acid amplification part 120e' is provided with a plurality of heating parts 121e, 122e, 123e, 121e', 122e', and 123e'.

Here, the heating parts 121e, 122e, and 123e constituting the first nucleic acid amplification part 120e respectively heat the plurality of heated areas with different temperatures to amplify the nucleic acid of the reaction sample. As in the first embodiment, the plurality of heated areas falls into an annealing area, an extension area, and a denaturation area. The heating parts 121e, 122e, and 123e include an annealing heating part 121e, an extension heating part 122e, and a denaturation heating part 123e respectively heating the annealing area AA, the extension area EA, and the denaturation area DA, to amplify the nucleic acid by polymerase chain reaction (PCR). Here, the first nucleic acid amplification part 120e is arranged in an order of the denaturation heating part 123e, the annealing heating part 121e, the extension heating part 122e, the annealing heating part 121e, the denaturation heating part 123e such that it is possible to amplify the flow in both directions.

Likewise, the heating parts 121e', 122e', 123e' constituting the second nucleic acid amplification part 120e' heat the plurality of heated areas with different temperatures to amplify the nucleic acid of the reaction sample. As in the first embodiment, the plurality of heated areas falls into an annealing area, an extension area, and a denaturation area. The heating parts 121e', 122e', and 123e' include an annealing heating part 121e', an extension heating part 122e', and a denaturation heating part 123e' respectively heating the annealing area AA, an extension area EA, and a denaturation area DA, to amplify the nucleic acid by PCR. Here, the second nucleic acid amplification part 120e' is arranged in an order of the denaturation heating part 123e', the annealing heating part 121e', the extension heating part 122e', the annealing heating part 121e', the denaturation heating part 123e' such that it is possible to amplify the flow in both directions.

With regard to the configuration described above, an operating process of the real-time nucleic acid amplification measuring apparatus 100e according to the second embodiment of the present invention will be described hereinbelow.

When the syringe pump applies pressure toward the second buffer chamber 117e in a state that the first buffer chamber 116e is filled with the reaction sample, the reaction sample flows to the microfluidic channel 111e from the first buffer chamber 116e. At this point, in the first nucleic acid amplification part 120e, the reaction sample is gradually heated by the heating parts 121e, 122e, and 123e such that nucleic acid is amplified.

The nucleic acid amplified by the first nucleic acid amplification part 120e is detected by the surface measurement part 130e, and the reaction sample is gradually heated again by the heating parts 121e', 122e', and 123e' in the second nucleic acid amplification part 120e' such that the nucleic acid is amplified. Then, the reaction sample flows to the second buffer chamber 117e.

When the syringe pump applies pressure toward the first buffer chamber 116e, the reaction sample flows to the microfluidic channel from the second buffer chamber 117e. At this point, in the second nucleic acid amplification part 120e', the reaction sample is heated gradually by the heating parts 121e', 122e', and 123e' such that the nucleic acid is amplified.

The nucleic acid amplified by the second nucleic acid amplification part 120e' is detected by the surface measurement part 130e, and the reaction sample is gradually heated again by the heating parts 121e, 122e, and 123e in first nucleic acid amplification part 120e such that the nucleic acid is amplified. Then, the reaction sample flows to the first buffer chamber 116e.

By repeating the above process, the surface measurement part 130e can perform the measurement of the nucleic acid amplified by the first nucleic acid amplification part 120e and the second nucleic acid amplification part 120e' in real-time.

Here, the gas discharging portion and the gas discharging vacuum portion 162 described in the first embodiment of the present invention may be applied to the second embodiment of the present invention. In addition, the configuration that the measurement area of the microfluidic channel 111, where the surface measurement part 130 detects nucleic acid, becomes narrow toward the surface measurement part 130 may be applied to the second embodiment of the present invention.

Meanwhile, in the real-time nucleic acid amplification measuring apparatuss 100 according to the first and second embodiment of the present invention, the measurement area where the surface measurement part 130 detects nucleic acid may be configured to be heated to a predetermined temperature.

In the first embodiment of FIG. 2, it is exemplified that the measurement area is provided in the annealing area AA heated by the annealing heating part 122. On the other hand, in the second embodiment of FIG. 11, it is exemplified that a separate heating part 124 is provided.

By heating the measurement area as described above, nucleic acid easily adheres to the surface measurement part 130 such as the metal thin film chip 133, whereby it is possible to perform the measurement of the nucleic acid more accurately.

Meanwhile, in the first embodiment described above, the examples of the fluid movement generating part 140 provided individually are described with reference to FIG. 4. In addition, when the microfluidic chip is disposed in the gravity direction such that the reaction sample in the microchannel channel flows in the gravity direction, a density change of the reaction sample heated by the heating parts 121, 122, and 123 arranged in the gravity direction causes heat convection whereby the reaction sample can circulate. That is, the plurality of heating parts 121, 122, and 123 constitute the fluid movement generating part 140.

Although the embodiments of the present invention have been disclosed for illustrative purposes, those skilled in the art will appreciate that various modifications, additions and substitutions are possible, without departing from the scope and spirit of the invention as disclosed in the accompanying claims. It is thus well known to those skilled in that the patent right of the present invention should be defined by the scope and spirit of the invention as disclosed in the accompanying claims. Accordingly, it should be understood that the present invention includes various modifications, additions and substitutions without departing from the scope and spirit of the invention as disclosed in the accompanying claims.

### <Description of reference numerals in the drawings>

100, 100e: real-time nucleic acid amplification measuring apparatus
110: microfluidic chip 111, 110e: microfluidic channel
112: upper surface 113: bottom surface
114: protruding part 116e: first sample buffer chamber
117e: second sample buffer chamber
121, 121e, 121e', 122, 122e, 122e', 123, 123e, 123e': heating part
130, 130a, 130c, 130d, 130e: surface measurement part
131, 131a, 131b: light source
131c: QCM sensor 131d: first electrode
132c: crystal resonator 132, 132a, 132b : light receiver
132d: second electrode 133, 133a : metal thin film chip
133b: nanoplasmonic sensor chip 133c: electrodes
133d: third electrode 134, 134a: coupler
134b: nano metal particle 134c: high frequency power supply
134d: measurement voltage supply 140: fluid movement generating part
141: impeller 142: impeller drive
143: interdigital transducer (IDT) 144: IDT power supply
145: dielectrophoretic electrodes 146: power supply for dielectrophoretic electrodes
150: sample injecting and closing part 151: sample inlet
152: sample outlet 153: four-way valve
154: gas discharging membrane 161: gas discharging portion
162: gas discharging vacuum portion

### Industrial Applicability

As described above, the present invention can be applied to the field of detecting the amplification process of nucleic acid in real-time, based on a surface measurement technique such as surface plasmon resonance (SPR).

## Claims

1. A real-time nucleic acid amplification measurement apparatus using a surface measurement technique, the apparatus comprising:
a microfluidic chip having a closed loop shaped-microfluidic channel;
a sample injecting and closing part communicating with the microfluidic channel, and operating in an injecting mode in which a reaction sample is injected to the microfluidic channel or operating in a closed mode in which the microfluidic channel forms a closed-loop in a state that the reaction sample has been injected to the microfluidic channel;
a fluid movement generating part inducing the reaction sample to circulate inside the microfluidic channel;
a plurality of heating parts individually heating a plurality of heating areas with different temperatures to amplify nucleic acid in the reaction sample; and
a surface measurement part detecting the nucleic acid in the reaction sample at a predetermined area inside the microfluidic channel.

2. A real-time nucleic acid amplification measurement apparatus using a surface measurement technique, the apparatus comprising:
a microfluidic chip provided with a microfluidic channel, and a first sample buffer chamber and a second sample buffer chamber, which are provided at both sides of the microfluidic channel respectively;
a fluid movement generating part inducing the reaction sample to oscillatingly flow in the first buffer chamber, the microfluidic channel, and the second buffer chamber;
a surface measurement part detecting nucleic acid in the reaction sample at a middle area of a flow direction in the microfluidic channel; and
a first nucleic acid amplification part and a second nucleic acid amplification part each provided at both sides of the surface measurement part and each provided with a plurality of heating parts individually heating a plurality of heated areas with different temperatures to amplify the nucleic acid of the reaction sample repeatedly flowing in the microfluidic channel.

3. The apparatus of claim 2, wherein the plurality of heated areas falls into a denaturation area, an annealing area, and an extension area, and
the heating parts include a denaturation heating part, an annealing heating part, and an extension heating part respectively heating the denaturation area, the annealing area, and the extension area to amplify nucleic acid by polymerase chain reaction (PCR).

4. The apparatus of claim 3, wherein the first nucleic acid amplification part and the second nucleic acid amplification part are arranged in an order of the denaturation heating part, the annealing heating part, the extension heating part, the annealing heating part, and the denaturation heating part.

5. The apparatus of claim 2, wherein the fluid movement generating part is provided in a type of a syringe pump.

6. The apparatus of claim 1, wherein the surface measurement part includes a surface plasmon resonance (SPR) sensing part using a SPR phenomenon.

7. The apparatus of claim 6, wherein the SPR sensing part includes:
a metal thin film chip provided at an inner surface of the microfluidic channel to detect plasmon reaction;
a coupler provided at outside the metal thin film chip;
a light source irradiating the metal thin film chip with measurement light from outside the microfluidic chip; and
a light receiver receiving a reflection light reflected by the metal thin film chip.

8. The apparatus of claim 7, wherein the SPR sensing part is provided with one method among a variable-angle SPR method, a variable-wavelength SPR method, and an SPR imaging method.

9. The apparatus of claim 1, wherein the surface measurement part includes:
a nanoplasmonic sensor chip provided at an inner surface of the microfluidic channel and on whose surface nano metal particles generating nanoplasmonic effect are solidified;
a light source irradiating the nanoplasmonic sensor chip with measurement light from outside the microfluidic channel to induce the nano metal particles to generate a nanoplasmonic effect; and
a light receiver receiving a light transmitted through the nanoplasmonic sensor chip,
wherein the surface measurement part detects nucleic acid by using at least one among a wavelength and an intensity of the transmitted light.

10. The apparatus of claim 1, wherein the surface measurement part includes:
a quartz crystal microbalance (QCM) sensor provided inside the microfluidic channel; and
a high frequency power supply applying high frequency power to the QCM sensor,
wherein the surface measurement part detects nucleic acid by using a frequency change according to a mass change of the QCM sensor due to nucleic acid adhered to a surface thereof.

11. The apparatus of claim 1, wherein the surface measurement part includes:
a first electrode provided at an inner surface of the microfluidic channel and having a characteristic that combines with nucleic acid;
a second electrode provided at the inner surface of the microfluidic channel and having a characteristic that does not combine with nucleic acid;
a third electrode applied with a different polarity with the first electrode and the second electrode; and
a measurement voltage supply applying same polarity of voltage to the first electrode and the second electrode, and applying voltage to the third electrode, the voltage having opposite polarity to the first electrode and the second electrode,
wherein the surface measurement part detects nucleic acid by using a current value according to a conductivity change while the measurement voltage supply alternately switches between positive voltage to negative voltage.

12. The apparatus of claim 1, wherein the plurality of heating parts falls into a denaturation area, an annealing area, and an extension area, and
the heating parts include a denaturation heating part, an annealing heating part, and an extension heating part respectively heating the denaturation area, the annealing area, and the extension area to amplify nucleic acid by polymerase chain reaction (PCR).

13. The apparatus of claim 1, wherein the sample injecting and closing part includes:
a four-way valve including a first connecting port, a second connecting port, a third connecting port, and a fourth connecting port, wherein the first connecting port and the second connecting port are connected to the microfluidic channel and the microfluidic channel forms the closed-loop if the first connecting port and the second connecting port are connected to each other;
a sample inlet having one end through which the reaction sample is injected and a remaining end is connected to the third connecting port of the four-way valve; and
a sample outlet having one end is connected to the fourth connecting port of the four-way valve;
wherein, in the injecting mode, the four-way valve connects the first connecting port and the third connecting port to each other and connects the second connecting port and the fourth connecting port such that the reaction sample injected through the sample inlet is allowed to flow into the microfluidic channel, and
in the closed mode, the four-way valve connects the first connecting port and the second connecting port to each other such that the microfluidic channel forms the closed-loop.

14. The apparatus of claim 13, wherein the sample injecting and closing part further includes a gas discharging membrane provided at an end of the sample outlet and allowing gas to pass therethrough and blocking liquid,
wherein the reaction sample injected into the microfluidic channel in the injecting mode passes the second connecting port, the microfluidic channel, and the fourth connecting port and flows to the sample outlet, and
gas inside the reaction sample is discharged to an outside of the microfluidic channel through the gas discharging membrane while being blocked by the gas discharging membrane.

15. The apparatus of claim 1, further comprising:
a gas discharging portion provided at least at a portion of an upper surface of the microfluidic channel in terms of the gravity direction, and made of a material that allows gas to pass therethrough and blocks liquid; and
a gas discharging vacuum portion applying a vacuum pressure to the gas discharging portion to discharge bubbles through the gas discharging portion, the bubbles generated in a heating process by the heating parts.

16. The apparatus of claim 1, wherein the plurality of heating parts constitutes the fluid movement generating part in which the microfluidic chip is disposed in the gravity direction such that the reaction sample in the microfluidic channel flows in the gravity direction,
the plurality of heating parts is disposed in the gravity direction and arranged in an order of decreasing temperature, and
a density change of the reaction sample heated by the plurality of the heating parts disposed in the gravity direction causes heat convection whereby the reaction sample circulates.

17. The apparatus of claim 1, wherein the fluid movement generating part includes:
a pump member made of an elastic material and constituting a wall surface of a area of the microfluidic channel; and
a pump drive pumping the pump member such that the reaction sample flows inside the microfluidic channel.

18. The apparatus of claim 1, wherein the fluid movement generating part includes:
an impeller operating to allow the reaction sample to flow in the microfluidic channel; and
an impeller drive disposed outside the microfluidic channel and driving the impeller by magnetic force.

19. The apparatus of claim 1, wherein the fluid movement generating part includes an acoustic wave generator generating a high-frequency sound wave in a flow direction of reaction sample to allow the reaction sample to flow.

20. The apparatus of claim 1, wherein the fluid movement generating part includes:
a plurality of dielectrophoretic electrodes arranged at an inner wall of the microfluidic channel in a flow direction of the reaction sample; and
a power supply for the dielectrophoretic electrodes, the power supply supplying power to cause a flow of the reaction sample by dielectrophoresis.

21. The apparatus of claim 20, wherein the fluid movement generating part further includes a laser emitting part irradiating one of the dielectrophoretic electrodes,
wherein the flow of the reaction sample and vortex occur in a direction starting from the irradiated dielectrophoretic electrode to another dielectrophoretic electrode.

22. The apparatus of claim 7, wherein the metal thin film chip is provided with a dextran-based or polymer-based three-dimensional surface material on a surface thereof to increase a sensing surface area, and provided with a receptor for reaction with nucleic acid on the three-dimensional surface material.

23. The apparatus of claim 22, wherein an inner wall surface of the microfluidic channel provided above the metal thin film chip is configured to protrude toward the metal thin film chip such that a portion of the channel provided with the metal thin film chip is configured to become narrow.

24. The apparatus of claim 23, further comprising:
a protruding part protruding toward the metal thin film chip from an inner wall surface of the microfluidic channel provided above the metal thin film chip such that a portion of the channel provided with the metal thin film chip is configured to become narrow,
wherein the protruding part is provided with a micro pattern inducing mix of the reaction sample flowing inside the microfluidic channel.
